# EUROPÄISCHE PATENTANMELDUNG

(11) **EP 4 749 272 A1**
(43) Veröffentlichungstag der Anmeldung: **27.05.2026**
(21) Anmeldenummer: 25215477.8
(22) Anmeldetag: 13.11.2025
(51) Int. Cl.: G01N 25/48, G06N 20/00

(54) **VERFAHREN UND SYSTEM ZUR RECHNERGESTÜTZTEN AUSWERTUNG DYNAMISCHER DIFFERENZKALORIMETRIEMESSDATEN**

(30) Priorität: 20.11.2024 DE 102024134082; 20.11.2024 US 202463722622 P
(71) Anmelder: Netzsch-Gerätebau GmbH, 95100 Selb (DE)
(72) Erfinder: Postel, Dr., Andreas, 95145 Oberkotzau (DE); Rudolph, Dr., Natalie, 97776 Eußenheim (DE)

(57) **Zusammenfassung**

Die vorliegende Erfindung schafft ein Verfahren zur rechnergestützten Auswertung dynamischer Differenzkalorimetriemessdaten. Ferner schafft die vorliegende Erfindung ein System zur rechnergestützten Auswertung dynamischer Differenzkalorimetriemessdaten sowie eine Verwendung eines derartigen Systems, insbesondere zur Rezyklatanalyse, zur analytischen Diskriminierung zwischen verschiedenen Kunststoffproben.

## Beschreibung

Die vorliegende Erfindung betrifft ein Verfahren und ein System zur rechnergestützten Auswertung dynamischer Differenzkalorimetriemessdaten, insbesondere zur analytischen Diskriminierung zwischen verschiedenen Kunststoffproben, beispielsweise im Rahmen einer Sortierung von Rezyklaten.

Die Wiederaufbereitung von Müll ist besonders im Bereich von Kunststoffabfällen wichtig, da deren Ablagerung in der Umwelt sich aufgrund der nahezu vollständigen biologischen Nichtabbaubarkeit unerwünscht ist. Die Wiederverwertung von Kunststoffen kann beispielsweise über ein Einschmelzen und Umformen von Kunststoffabfällen erfolgen. Damit diese Art von Recycling effizient ist, werden Kunststoffabfälle vor der Wiederaufbereitung üblicherweise nach deren Materialeigenschaften wie beispielsweise Farbe und/oder Polymertyp sortiert. Dieses Sortieren erfordert eine Untersuchung der Zusammensetzung der Kunststoffabfälle, deren Durchführung wegen der Unvorhersagbarkeit des eingehenden Materialstroms häufig zeitaufwändig und daher kostenintensiv ist.

Der Grad der Qualitätsbestimmung und Qualitätssicherung bei der Charakterisierung der Hochwertigkeit von Polymerrezyklaten hängt von der Qualität der eingesetzten Separierungs- und Disl<riminierungsverfahren zur Bearbeitung der Ausgangsstoffe ab. Insbesondere die physikalischen Eigenschaften der Polymerrezyklate müssen zur effizienten Wiederverwertung innerhalb enger Toleranzen spezifiziert und garantiert werden können. Moderne rechnergestützte Datenverarbeitung der Messdaten effizienter Messgeräte kann die Qualitätsanalyse von Polymerrezyklaten beschleunigen und zuverlässiger machen. Beispiele für Systeme und Verfahren zum Klassifizieren und Sortieren von Kunststoffmaterialien unter Verwendung eines Bildverarbeitungssystems und eines oder mehrerer Sensorsysteme werden in der Druckschrift US 2022/0161298 A1 erläutert.

Das Messprinzip der thermischen Analyse, und dabei insbesondere das physikalische Messprinzip der dynamischen Differenzkalorimetrie ("differential scanning calorimetry", DSC), kann bei der experimentellen Charakterisierung von Kunststoffabfällen variabler Qualität helfen. DSC-Messgeräte messen die Wärmekapazität einer Probe über die Aufzeichnung der Wärmeflussrate in die Probe verglichen mit einer Referenzprobe. Aus der dadurch gewonnenen Auftragung des Wärmeflusses gegenüber der Probentemperatur bzw. deren zeitlichem Verlauf können Materialübergangspunkte wie beispielsweise eine Glasübergangstemperatur oder eine Schmelztemperatur, ein Kristallinitätsgrad thermoplastischer Matrizen, oder ein Aushärteverhalten bzw. eine Restreaktionswärme duroplastischer Materialien bestimmt werden.

Die Druckschrift WO 2022/170273 A1 offenbart Systeme und Verfahren zum Klassifizieren und Sortieren von verschiedenfarbiger Kunststoffmaterialien unter Verwendung eines Bildverarbeitungssystems oder eines oder mehrerer Sensorsysteme, wobei die erfassten Bilddaten in einem maschinellen Lernsystem verarbeitet werden, um jedes der Materialien zu Zwecken der Sortierung zu identifizieren oder zu klassifizieren. Die Druckschrift EP 4 209 781 A1 offenbart computer-implementierte Verfahren zur thermischen Analyse von Materialproben.

Xin Lv, Shuyu Wang, Peng Shan, Yuliang Zhao, und Lei Zuo: "A machine learning based method for automatic differential scanning calorimetric signal analysis", Measurement, vol. 187, 110218, 2022 offenbart rechnergestützte Auswerteverfahren für DSC-Messdaten auf der Basis von semi-automatisierten Maschinenlernmodellen. Amir Bashirgonbadi, Yannick Ureel, Laurens Delva, Rudinei Fiorio, Kevin M. Van Geem, und Kim Ragaert: "Accurate determination of polyethylene (PE) and polypropylene (PP) content in polyolefin blends using machine learning-assisted differential scanning calorimetry (DSC) analysis", Polymer Testing, vol. 131, 108353, Februar 2024 offenbart den Einsatz von künstlicher Intelligenz bei der Auswertung von DSC-Messkurven in der Charakterisierung von Polymerrezyklaten.

Es ist Aufgabe der vorliegenden Erfindung, Möglichkeiten zur kostengünstigeren, einfacheren und schnelleren Analyse von DSC-Messdaten zu schaffen. Insbesondere ist es eine Aufgabe der vorliegenden Erfindung, die analytische Diskriminierung zwischen verschiedenen Kunststoffproben, beispielsweise bei der Klassifikation und Quantifizierung von Rezyklaten, zu beschleunigen und zu verbessern.

Erfindungsgemäß wird diese Aufgabe jeweils durch die Gegenstände der unabhängigen Ansprüche gelöst.

Vorteilhafte Ausführungsformen und Weiterbildungen ergeben sich aus den auf die unabhängigen Ansprüche rückbezogenen Unteransprüchen sowie aus der Beschreibung unter Bezugnahme auf die Figuren.

Die obigen Ausführungsformen und Weiterbildungen lassen sich, sofern sinnvoll, beliebig miteinander kombinieren. Weitere mögliche Ausführungsformen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

Die vorliegende Erfindung wird nachfolgend anhand von Ausführungsbeispielen unter Bezugnahme auf die beiliegenden Figuren der Zeichnungen näher erläutert. Von den Figuren zeigen:
- Fig. 1: ein Ablaufdiagramm eines Verfahrens zur rechnergestützten Auswertung dynamischer Differenzkalorimetriemessdaten gemäß einem Ausführungsbeispiel der Erfindung;
- Fig. 2: eine schematische Darstellung eines Ausführungsbeispiels eines Systems zur rechnergestützten Auswertung dynamischer Differenzkalorimetriemessdaten, insbesondere zur Implementierung des Verfahrens gemäß dem Ausführungsbeispiel nach Fig. 1; und
- Fig. 3: eine schematische Darstellung einer Rezyklatsortiervorrichtung gemäß einem Ausführungsbeispiel der Erfindung, welche ein System zur rechnergestützten Auswertung dynamischer Differenzkalorimetriemessdaten aufweist.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

Obwohl vorliegend spezifische Ausführungsformen und Weiterbildungen dargestellt und beschrieben sind, wird der Fachmann bevorzugen, dass eine Vielzahl von alternativen und/oder gleichartigen Ausführungen die dargestellten und beschriebenen spezifischen Ausführungsbeispiele ersetzen können, ohne vom Umfang der vorliegenden Erfindung abzukehren. Diese Anmeldung soll allgemein alle Abwandlungen oder Änderungen der hierin beschriebenen spezifischen Ausführungsbeispiele abdecken.

Die beiliegenden Figuren sollen ein weiteres Verständnis von Ausführungsformen der Erfindung vermitteln und dienen im Zusammenhang mit der Beschreibung der Erklärung von Prinzipien und Konzepten der Erfindung. Andere Ausführungsbeispiele und viele der genannten Vorteile ergeben sich im Hinblick auf die Zeichnungen. Die Zeichnungen sind lediglich als schematische Zeichnungen zu verstehen und die Elemente der Zeichnungen sind nicht notwendigerweise maßstabsgetreu zueinander dargestellt. Richtungsangebende Terminologie wie etwa "oben", "unten", "links", "rechts", "über", "unter", "horizontal", "vertikal", "vorne", "hinten" und ähnliche Angaben werden lediglich zu erläuternden Zwecken verwendet und dienen nicht der Beschränkung der Allgemeinheit auf spezifische Ausgestaltungen wie in den Figuren gezeigt.

Gestrichelte Linien in den Figuren der Zeichnungen verdeutlichen, dass die Verbindungen zwischen den die gestrichelten Linien verbindenden Komponenten nicht zwingend physischen Kontakt miteinander haben müssen, sondern gleichermaßen drahtlos zueinander gekoppelt sein können.

In der folgenden Beschreibung wird auf selbstlernende Algorithmen Bezug genommen, welche in einem System künstlicher Intelligenz (Kl-System) genutzt werden. Allgemein gesprochen, bildet ein selbstlernender Algorithmus kognitive Funktionen nach, die nach menschlichem Ermessen einer menschlichen Denkleistung zugeordnet werden. Dabei kann der selbstlernende Algorithmus durch Hinzunahme neuer Trainingsinformationen die bislang aus alten Trainingsinformationen gewonnenen Erkenntnisse dynamisch an die veränderten Umstände anpassen, um in der Gesamtheit der Trainingsinformationen Muster und Gesetzmäßigkeiten zu erkennen und zu extrapolieren.

In selbstlernenden Algorithmen im Sinne der vorliegenden Erfindung können alle Arten des den menschlichen Erkenntnisgewinn bildenden Trainings verwendet werden, wie beispielsweise überwachtes Lernen, teil-überwachtes Lernen, selbständiges Lernen auf der Basis generativer, nicht-generativer oder tiefer kontradiktorischer Netzwerke ("adversarial networks", AN), bestärkendes Lernen oder aktives Lernen. Dabei kann in jeder Instanz merkmalsbasiertes Lernen ("representation learning") eingesetzt werden. Die selbstlernenden Algorithmen im Sinne der vorliegenden Erfindung können insbesondere eine iterative Anpassung von zu lernenden Parametern und Merkmalen über rückkoppelnde Analyse vornehmen.

Ein selbstlernender Algorithmus im Sinne der vorliegenden Erfindung kann auf Regressoren, einem Stützvektorklassifikator ("support vector network", SVN), einem neuronalen Netzwerk wie etwa ein faltendes neuronales Netzwerk ("convolutional neural network", CNN), ein Kohonen-Netz, ein rekurrentes neuronales Netzwerk, ein zeitverzögerndes neuronales Netzwerk ("time-delayed neural network", TDNN) oder ein oszillierendes neuronales Netzwerk ("oscillatory neural network", ONN), einem Random-Forest-Klassifikator, einem Entscheidungsbaum-Klassifikator, einem Monte-Carlo-Netzwerk oder einem Bayes'schen Klassifikator aufbauen. Dabei kann ein selbstlernender Algorithmus im Sinne der vorliegenden Erfindung eigenschaftshereditäre Algorithmen, k-Means-Algorithmen wie etwa Lloyd- oder MacQueen's-Algorithmen oder TD-Lernalgorithmen wie etwa SARSA oder Q-Learning einsetzen.

Differenzkalorimetriemessdaten im Sinne der vorliegenden Erfindung können insbesondere alle Datensätze umfassen, welche von Differenzkaloriemetriemessgeräten bzw. Differenzkaloriemetriesensoren erzeugt werden. Ein Differenzkaloriemetriemessgerät ist ein Gerät, das verwendet wird, um den Wärmestrom durch verschiedene Substanzen zu messen, insbesondere in Kunststoffproben. Das Gerät arbeitet auf der Grundlage der Differenzkalorimetrie-Technologie ("differential scanning calorimetry", DSC), die darauf abzielt, die Menge an Wärme zu messen, die bei einer Enthalpieänderung zwischen einer Probe und einer Referenzsubstanz frei wird.

Dabei wird eine Probe, beispielsweise ein Stück Kunststoff in einer speziellen Kammer platziert, die neben einer leeren Referenzkammer liegt. Die beiden Kammern werden isoliert und gleichmäßig erhitzt, beispielsweise über eine Wärmematte unter den Kammern, die für eine kontinuierliche und vordefinierte Wärmezufuhr sorgt. Aufgrund der Wärmekapazität der Probe kommt es hierbei zu endothermen oder exothermen Prozessen sowie Phasenübergängen an Schmelz- oder Sublimationspunkten, was Aufschluss über Wärmeströme in Abhängigkeit der Temperatur gibt. Die Temperaturänderung beim kontrollierten Aufheizen und Abkühlen in beiden Kammern wird zeitaufgelöst durch Wärmefühler gemessen, die an jeder Kammer angebracht sind. Differenzkalorimetriemessungen liefern Informationen über Kennwerte zur Charakterisierung thermischer Eigenschaften der Kunststoffprobe, wie zum Beispiel Glasübergangstemperaturen, Schmelzpunkte, Rekationsenthalpien, Kristallinitätsgrade sowie spezifische Wärmekapazitäten.

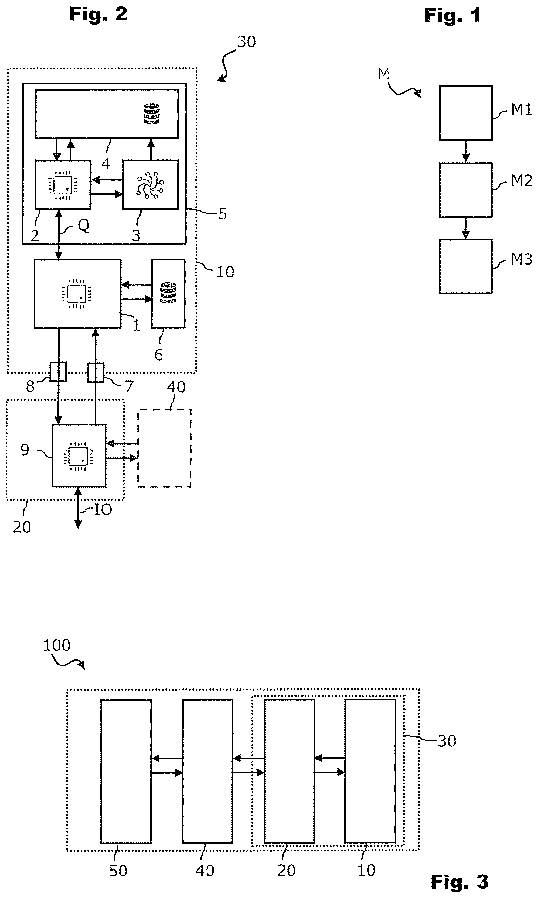

Fig. 1 zeigt ein Ablaufdiagramm eines beispielhaften Verfahrens M zur rechnergestützten Auswertung dynamischer Differenzkalorimetriemessdaten. Das Verfahren M kann insbesondere unter Verwendung eines Systems zur rechnergestützten Auswertung dynamischer Differenzkalorimetriemessdaten implementiert werden, beispielsweise des Systems 30 wie beispielhaft in Fig. 2 gezeigt. Das Verfahren M kann zum Beispiel zur analytischen Diskriminierung zwischen verschiedenen Kunststoffproben, beispielsweise bei der Sortierung von Rezyklaten in einer Rezyklatsortiervorrichtung 100 wie beispielhaft in Fig. 3 dargestellt, eingesetzt werden.

In einem ersten Schritt M1 des Verfahrens M erfolgt ein Erzeugen einer DSC-Messkurve einer unbekannten Probe durch eine DSC-Messeinrichtung 40. In einem zweiten Schritt M2 ermittelt ein KI-System 10 ein Datenmuster in der DSC-Messkurve durch Einsatz selbstlernender Algorithmen. Schließlich werden auf der Basis der ermittelten Datenmuster in einem Schritt M3 durch das KI-System 10 die enthaltenen Materialklassen in der DSC-Messkurve zu bestimmten Materialklassen oder bestimmten Materialien zugewiesen.

Fig. 2 zeigt eine beispielhafte Illustration eines Systems 30 zur rechnergestützten Auswertung dynamischer Differenzkalorimetriemessdaten (DSC-Messdaten). Das System 30 kann insbesondere zur Implementierung des Verfahrens M der Fig. 1 eingesetzt werden.

Das System 30 umfasst ein KI-System 10 und ein Steuersystem 20 für eine Differenzkaloriemetriemesseinrichtung (DSC-Messeinrichtung) 40. Das Steuersystem 20 weist einen Steuerprozessor 9 auf, welcher über eine Eingabeschnittstelle 7 und eine Ausgabeschnittstelle 8 mit dem KI-System 10 einerseits und mit der DSC-Messeinrichtung 40 andererseits gekoppelt ist.

Das Steuersystem 20 dient der Steuerung der DSC-Messeinrichtung 40, indem der Steuerprozessor 9 ein Steuerprogramm für die DSC-Messeinrichtung 40 vorgibt. Beispielsweise kann das Steuersystem 20 die Protokollparameter für eine DSC-Messung vorgeben, wie etwa Aufheizraten oder Abkühlraten für die Probe, eine Anzahl der durchzuführenden Heizzyklen, Wartezeiten zwischen Heiz- und Abkühlphasen oder dergleichen.

Über die Eingabeschnittstelle 7 können DSC-Messdaten von dem Steuerprozessor 9 in das KI-System 10 eingegeben werden. Diese DSC-Messdaten können beispielsweise anhand von bekannten oder unbekannten Proben gewonnene DSC-Messkurven oder von angeschlossenen weiteren Systemen als computergenerierte Trainingsdaten D an den Steuerprozessor 9 übergebene Daten sein.

Das KI-System 10 weist einen Datenanalyseprozessor 1 sowie ein Maschinenlernsystem 5 (ML-System) auf. Das ML-System 5 wiederum weist einen Kl-Prozessor 2, einen auf selbstlernenden Algorithmen basierenden Regelwerkgenerator 3 und einen Referenzregelwerkspeicher 4 auf. Das KI-System 10 steht über den Datenanalyseprozessor 1 mit dem Steuerprozessor 9 in bidirektionaler Datenkommunikation. Der Steuerprozessor 9 kann dem KI-System 10 zunächst DSC-Messkurven als Basis-Trainingsdaten bereitstellen. Diese Basis-Trainingsdaten können als Basis für die Detektion von Mustern und Gesetzmäßigkeiten in dem Kurvenverlauf der DSC-Messkurven durch den Regelwerkgenerator 3 dienen. Der Regelwerkgenerator 3 kann beispielsweise Regressoren, einen Stützvektorklassifikator, ein neuronales Netzwerk, einen Random-Forest-Klassifikator, einen Entscheidungsbaum-Klassifikator, ein Monte-Carlo-Netzwerk oder einen Bayes'schen Klassifikator aufweisen.

Die detektierten Muster und Gesetzmäßigkeiten in den Kurvenverläufen der DSC-Messkurven werden zunächst iterativ in einem Trainingsregelwerk abgelegt, welches dynamisch und laufend aktualisiert wird. Aus dem Trainingsregelwerk wird ein operatives Referenzregelwerk gebildet, welches der Regelwerkgenerator 3 in den Referenzregelwerkspeicher 4 speichert. Wenn der Kl-Prozessor 2 eine Anfrage Q des Datenanalyseprozessors 1 erhält, Datenmuster in DSC-Messkurven unbekannter Proben zu ermitteln, greift der Kl-Prozessor 2 als Referenz auf das in dem Referenzregelwerkspeicher 4 abgelegte Referenzregelwerk zurück. Gegenüber dieser Referenz überprüft der Kl-Prozessor 2, mit welcher Zuweisung zu bestimmten Materialklassen oder bestimmten Materialien eine möglichst gute Übereinstimmung mit den in der erhaltenen DSC-Messkurve enthaltenen Materialklassen existiert. Der Regelwerkgenerator 3 kann das in dem Referenzregelwerkspeicher 4 abgelegte Referenzregelwerk in periodischen Abständen auf der Basis neu hinzukommender DSC-Messdaten oder auf der Basis neuer externer Vorgaben aktualisieren.

Die Ergebnisse der Datenmusterermittlung werden von dem KI-Prozessor 2 an den Datenanalyseprozessor 1 rückübermittelt. Der Datenanalyseprozessor 1 kann dann über die Ausgabeschnittstelle 8 ein Analyseergebnis an den Steuerprozessor 9 ausgeben, welches angibt, welche Materialklasse bzw. welches Material der der von dem Steuerprozessor 9 erhaltenen DSC-Messkurve zugrunde liegenden unbekannten Probe zuzuordnen ist.

Das KI-System 10 kann zudem eine Steuerprogrammdatenbank 6 aufweisen, welche mit dem Datenanalyseprozessor 1 gekoppelt ist. Wenn der Datenanalyseprozessor 1 von dem Steuerprozessor 9 eine DSC-Messkurve erhält, deren Zuweisung zu einer bestimmten Materialklasse oder einem bestimmten Material durch den Kl-Prozessor 2 nur unzureichend bzw. nur mit einer Konfidenz unterhalb einer einstellbaren Konfidenzschwelle möglich ist, kann der Datenanalyseprozessor 1 dem Steuerprozessor 9 eine Änderung des von dem Steuersystem 20 vorgebbaren Steuerprogramms vorschlagen. Beispielsweise kann der Kl-Prozessor 2 bei einer unzureichenden Klassifizierungsmöglichkeit der Materialklasse oder des Materials auf der Basis einer einer Anfrage Q zugrunde liegenden DSC-Messkurve Indikationen angeben, welche Teile der DSC-Messkurve die Klassifizierung erschweren. Der Datenanalyseprozessor 1 kann auf der Basis der durch den KI-Prozessor 2 angegebenen Teile der DSC-Messkurve aus der Steuerprogrammdatenbank 6 Steuerprogramme auswählen, deren Protokollparameter für eine DSC-Messung gegenüber dem bisher durch das Steuersystem 20 vorgegebenen Steuerprogramm derart geändert sind, dass eine erneute DSC-Messung substantielle Änderungen in den problematischen Teilen der DSC-Messkurve erwarten lässt.

Die Komponenten des KI-Systems 10 können zusammen mit dem Steuersystem 20 in einer lokalen Datenverarbeitungsanlage installiert sein. Es kann jedoch auch möglich sein, einzelne Komponenten oder Systembestandteile außerhalb der lokalen Datenverarbeitungsanlage zu installieren. Beispielsweise kann es möglich sein, das KI-System 10 in einer Cloud-Umgebung zu betreiben, so dass die Eingabeschnittstelle 7 und die Ausgabeschnittstelle 8 über Fernzugriffsnetzwerke wie beispielsweise das Internet realisiert werden können.

Weiterhin kann der Steuerprozessor 9 eine Eingabe-/Ausgabeschnittstelle aufweisen, über die Eingaben und Ausgaben IO durch einen Nutzer des Systems 30 getätigt werden können.

Fig. 3 zeigt eine beispielhafte Implementierung eines Systems 30 zur rechnergestützten Auswertung dynamischer Differenzkalorimetriemessdaten in einer Rezyklatsortiervorrichtung 100. Die Rezyklatsortiervorrichtung 100 umfasst beispielsweise ein automatisches Sortiersystem 50 für Kunststoffe. Das automatische Sortiersystem 50 ist mit einer DSC-Messeinrichtung 40 gekoppelt, welche dazu ausgelegt ist, von unbekannten Kunststoffproben, die in dem automatischen Sortiersystem 50 verarbeitet werden, DSC-Messkurven aufzuzeichnen. Die aufgezeichneten DSC-Messkurven werden an ein Steuersystem 20 übergeben, welches diese wiederum an ein KI-System 10 zur automatischen Zuordnung eines bestimmten Materials oder einer bestimmten Materialklasse zu den in den aufgezeichneten DSC-Messkurven enthaltenen Materialklassen vornimmt. Das Steuersystem 20 und das KI-System 10 können beispielsweise wie im Zusammenhang mit Fig. 2 erläutert betrieben werden.

Das Steuersystem 20 dient ferner der Steuerung der DSC-Messeinrichtung 40, insbesondere zur Vorgabe von Protokollparametern für eine DSC-Messung der DSC-Messeinrichtung 40, wie etwa Aufheizraten oder Abkühlraten für die Probe, eine Anzahl der durchzuführenden Heizzyklen, Wartezeiten zwischen Heiz- und Abkühlphasen oder dergleichen.

In der vorangegangenen detaillierten Beschreibung sind verschiedene Merkmale zur Verbesserung der Stringenz der Darstellung in einem oder mehreren Beispielen zusammengefasst worden. Es sollte dabei jedoch klar sein, dass die obige Beschreibung lediglich illustrativer, keinesfalls jedoch beschränkender Natur ist. Sie dient der Abdeckung aller Alternativen, Modifikationen und Äquivalente der verschiedenen Merkmale und Ausführungsbeispiele. Viele andere Beispiele werden dem Fachmann aufgrund seiner fachlichen Kenntnisse in Anbetracht der obigen Beschreibung sofort und unmittelbar klar sein.

Die Ausführungsbeispiele wurden ausgewählt und beschrieben, um die der Erfindung zugrundeliegenden Prinzipien und ihre Anwendungsmöglichkeiten in der Praxis bestmöglich darstellen zu können. Dadurch können Fachleute die Erfindung und ihre verschiedenen Ausführungsbeispiele in Bezug auf den beabsichtigten Einsatzzweck optimal modifizieren und nutzen. In den Ansprüchen sowie der Beschreibung werden die Begriffe "beinhaltend" und "aufweisend" als neutralsprachliche Begrifflichkeiten für die entsprechenden Begriffe "umfassend" verwendet. Weiterhin soll eine Verwendung der Begriffe "ein", "einer" und "eine" eine Mehrzahl derartig beschriebener Merkmale und Komponenten nicht grundsätzlich ausschließen.

### Bezugszeichenliste

- 1: Datenanalyseprozessor
- 2: Kl-Prozessor
- 3: Regelwerkgenerator
- 4: Referenzregelwerkspeicher
- 5: Maschinenlernsystem
- 6: Steuerprogrammdatenbank
- 7: Eingabeschnittstelle
- 8: Ausgabeschnittstelle
- 9: Steuerprozessor
- 10: KI-System
- 20: Steuersystem
- 30: System
- 40: DSC-Messeinrichtung
- 50: automatisches Sortiersystem
- 100: Rezyklatsortiervorrichtung
- Q: Anfrage
- IO: Eingaben/Ausgaben
- M: Verfahren zur rechnergestützten Auswertung dynamischer Differenzkalorimetriemessdaten
- M1-M5: Verfahrensschritte

## Patentansprüche

1. System (30) zur rechnergestützten Auswertung dynamischer Differenzkalorimetriemessdaten, DSC-Messdaten, umfassend:
ein Steuersystem (20) für eine DSC-Messeinrichtung (40), mit einem Steuerprozessor (9), welcher dazu ausgelegt ist, DSC-Messkurven unbekannter Proben von der DSC-Messeinrichtung (40) zu empfangen; und
ein KI-System (10) mit einem Datenanalyseprozessor (1) und einem Maschinenlernsystem (5), wobei das KI-System (10) über den Datenanalyseprozessor (1) mit dem Steuerprozessor (9) in bidirektionaler Datenkommunikation steht und dazu ausgelegt ist, Datenmuster in den DSC-Messkurven unbekannter Proben durch Einsatz selbstlernender Algorithmen zu erkennen und auf der Basis der ermittelten Datenmuster eine Zuweisung der in den DSC-Messkurven enthaltenen Materialklassen zu bestimmten Materialklassen oder bestimmten Materialien zu treffen.

2. System (30) nach Anspruch 1, wobei das Maschinenlernsystem (5) einen Kl-Prozessor (2), einen auf selbstlernenden Algorithmen basierenden Regelwerkgenerator (3) und einen Referenzregelwerkspeicher (4) aufweist.

3. System (30) nach Anspruch 2, wobei der Regelwerkgenerator (3) Regressoren, einen Stützvektorklassifikator, ein neuronales Netzwerk, einen Random-Forest-Klassifikator, einen Entscheidungsbaum-Klassifikator, ein Monte-Carlo-Netzwerk oder einen Bayes'schen Klassifikator aufweist.

4. System (30) nach einem der Ansprüche 1 bis 3, wobei der Steuerprozessor (9) ferner dazu ausgelegt ist, der DSC-Messeinrichtung (40) ein Steuerprogramm vorzugeben, in dem Werte für Protokollparameter für eine DSC-Messung durch die DSC-Messeinrichtung (40) vorgegeben werden können.

5. System (30) nach Anspruch 4, wobei das KI-System (10) eine Steuerprogrammdatenbank (6) aufweist, welche mit dem Datenanalyseprozessor (1) gekoppelt ist, und in welcher verschiedene Steuerprogramme für die DSC-Messeinrichtung (40) gespeichert sind.

6. System (30) nach Anspruch 5, wobei der Datenanalyseprozessor (1) dazu ausgelegt ist, aus der Steuerprogrammdatenbank (6) Steuerprogramme auszuwählen, deren Protokollparameter für eine DSC-Messung gegenüber dem bisher durch das Steuersystem (20) vorgegebenen Steuerprogramm geändert sind, falls der Datenanalyseprozessor (1) von dem Steuerprozessor (9) eine DSC-Messkurve erhält, deren Zuweisung zu bestimmten Materialklassen oder bestimmten Materialien durch den KI-Prozessor (2) nur unzureichend bzw. nur mit einer Konfidenz unterhalb einer einstellbaren Konfidenzschwelle möglich ist.

7. System (30) nach einem der Ansprüche 1 bis 6, wobei das KI-System (10) in einer Cloud-Umgebung implementiert ist.

8. Verfahren (M) zur rechnergestützten Auswertung dynamischer Differenzkalorimetriemessdaten, DSC-Messdaten, umfassend:
Erzeugen (M1) einer oder mehrerer DSC-Messkurven einer unbekannten Probe durch eine DSC-Messeinrichtung (40);
Ermitteln (M2), durch ein KI-System (10), eines Datenmusters in den DSC-Messkurven durch Einsatz selbstlernender Algorithmen; und
Zuweisen (M3), auf der Basis der ermittelten Datenmuster, der in den DSC-Messkurven enthaltenen Materialklassen zu bestimmten Materialklassen oder bestimmten Materialien durch das KI-System (10).

9. Verfahren (M) nach Anspruch 8, wobei das KI-System (10) ein Maschinenlernsystem (5) aufweist, welches einen KI-Prozessor (2), einen auf selbstlernenden Algorithmen basierenden Regelwerkgenerator (3) und einen Referenzregelwerkspeicher (4) aufweist.

10. Verfahren (M) nach Anspruch 9, wobei der Regelwerkgenerator (3) Regressoren, einen Stützvektorklassifikator, ein neuronales Netzwerk, einen Random-Forest-Klassifikator, einen Entscheidungsbaum-Klassifikator, ein Monte-Carlo-Netzwerk oder einen Bayes'schen Klassifikator aufweist.

11. Verfahren (M) nach einem der Ansprüche 8 bis 10, wobei die DSC-Messeinrichtung (40) die DSC-Messkurve gemäß einem Steuerprogramm erzeugt, in dem Werte für Protokollparameter für eine DSC-Messung vorgegeben sind.

12. Verfahren (M) nach Anspruch 11, das KI-System (10) eine Steuerprogrammdatenbank (6) aufweist, welche mit dem Datenanalyseprozessor (1) gekoppelt ist, und in welcher verschiedene Steuerprogramme für die DSC-Messeinrichtung (40) gespeichert sind.

13. Verfahren (M) nach Anspruch 12, wobei aus der Steuerprogrammdatenbank (6) Steuerprogramme ausgewählt werden, deren Protokollparameter für eine DSC-Messung gegenüber dem bisher durch das Steuersystem (20) vorgegebenen Steuerprogramm geändert sind, falls das Zuweisen (M3) zu bestimmten Materialklassen oder bestimmten Materialien durch das KI-System (10) nur unzureichend bzw. nur mit einer Konfidenz unterhalb einer einstellbaren Konfidenzschwelle möglich ist.

14. Verwendung eines Systems (30) nach einem der Ansprüche 1 bis 7 zur analytischen Diskriminierung zwischen verschiedenen Kunststoffproben.

15. Rezyklatsortiervorrichtung (100), umfassend eine Differenzkalorimetriemesseinrichtung, DSC-Messeinrichtung (40), und ein System (30) zur rechnergestützten Auswertung dynamischer DSC-Messdaten gemäß einem der Ansprüche 1 bis 7.
